# EUROPEAN PATENT SPECIFICATION

(11) **EP 2 840 938 B1**
(45) Date of publication and mention of the grant of the patent: **20.09.2017**
(21) Application number: 13781248.3
(22) Date of filing: 26.04.2013
(51) Int. Cl.: A47G 9/10, A61F 5/37, A61G 7/07

(54) **THERAPEUTIC PILLOW**
THERAPEUTISCHES KISSEN
COUSSIN THÉRAPEUTIQUE

(30) Priority: 27.04.2012 US 201261639587 P; 07.09.2012 US 201213606693
(43) Date of publication of application: 04.03.2015
(73) Proprietor: Marinkovic, John, Paramus, NJ 07652 (US)
(72) Inventor: Marinkovic, John, Paramus, NJ 07652 (US)
(74) Representative: Chapman, Paul Gilmour
(86) International application number: PCT/US2013/038349
(87) International publication number: WO 2013/163507

(56) References cited:
- WO-A1-98/07358
- WO-A1-2005/032309
- CA-A1- 2 431 656
- FR-A1- 2 821 733
- RU-U1- 56 142
- US-A- 5 926 879
- US-A- 5 940 913
- US-A1- 2011 231 999
- US-B1- 6 823 546

## Description

### TECHNICAL FIELD

This invention relates to pillows or cushions and more particularly, to custom, therapeutic pillows or cushions, designed to be placed under the head and neck of a patient lying in a supine or side-supported position.

### BACKGROUND

Because approximately one-third of all human existence is spent in a supine position, there has been great interest in developing pillows or cushions that properly support a person's body in such position. Some pillows are marketed as being posture or cervical pillows that are designed to support the head and spine, and in particular, the neck vertebrae, in the most normal, comfortable and unstressed position, thereby aiding in relieving stress in the cervical or neck portion of the upper spine, and for promoting proper posture.

The neck of a person lying in a supine or sidelying position is often out of alignment with the person's spine. This is commonly the case when the person's neck is supported by a pillow of multiple pillows such that the neck lies at an angle defined by the deflected height of the pillow(s) and this angle is typically not co-planar with the spine. The deflected height of the pillow is closely related to its stiffness, which is conventionally provided by filling material disposed within a fabric covering. Conventional filling material can include feathers, cotton, or a synthetic filler.

Recently, a number of pillows have been formed of viscoelastic material, such as a viscoelastic foam material that forms the pillow. These types of pillows are often referred to as memory foam pillows. The viscoelastic foam responds to changes in temperature such that body heat molds the pillow to conform to the curves of a body for comfort and support. This allows the shape of the pillow to more closely follow the contours of the body and to promote an improved alignment of the neck and spine when a person is in a supine of sidelying position. While these pillows provide comfort and some therapeutic effect, they often do not promote optimal alignment of the neck and spine. There is therefore a need to provide an improved pillow that not only is comfortable but also provides a therapeutic effect and promotes improved alignment of the neck and spine when a person is in a supine or sidelying position. Pillows are known from US 6 823 546 B1 and US 5 926 879 A.

### SUMMARY

In accordance with the present invention, there is provided a pillow according to claim 1. Certain preferred and optional features of the invention are set out in the dependent claims. Other aspects, features and advantages of the invention will be apparent in view of the accompanying description of certain embodiments thereof when considered in connection with the accompanying drawing figures.

### BRIEF DESCRIPTION OF THE DRAWINGS

Fig. 1 is a side elevation view of a pillow in use in a first sleep position;
Fig. 2 is a perspective view thereof;
Fig. 3 is a side elevation view thereof;
Fig. 4 is a top plan view thereof;
Fig. 5 is a front elevation view thereof;
Fig. 6 is a perspective view of a pillow according to the invention in use in a second sleep position;
Fig. 7 is a side elevation view thereof;
Fig. 8 is a top plan view thereof;
Fig. 9 is a front elevation view thereof;
Fig. 10 is a side elevation view of a pillow according to the invention;
Fig. 11 is a perspective view thereof showing a two part construction embodiment;
Fig. 12 is a perspective view thereof showing a single unitary construction embodiment;
Fig. 13 is a top plan view thereof;
Fig. 14 is a front elevation view thereof;
Fig. 15 is a front elevation view showing another pillow;
Fig. 16 is a front and side perspective view of another pillow;
Fig. 17 is a front elevation view of the pillow of Fig. 16;
Fig. 18 is a rear elevation view of the pillow of Fig. 16;
Fig. 19 is a side elevation view of the pillow of Fig. 16;
Fig. 20 is a top plan view of the pillow of Fig. 16;
Fig. 21 is a bottom plan view of the pillow of Fig. 16;
Fig. 22 is a front and side perspective view of a pillow according to the invention;
Fig. 23 is a front elevation view of the pillow of Fig. 22;
Fig. 24 is a rear elevation view of the pillow of Fig. 22;
Fig. 25 is a side elevation view of the pillow of Fig. 22;
Fig. 26 is a top plan view of the pillow of Fig. 22; and
Fig. 27 is a bottom plan view of the pillow of Fig. 22.
It is to be noted that the pillows shown in figures 1-5 and 16-21 do not represent embodiments according to the invention.

### DETAILED DESCRIPTION OF CERTAIN EMBODIMENTS

In general, the present invention provides an orthopedic (therapeutic) pillow having advantages over other such pillows known and used in the art. Past orthopedic pillows often have a peanut shape or other such configuration that for a majority of people is uncomfortable. While sleeping on one's side, such prior art pillows typically cause the neck to arch over excessively laterally, in turn creating pressure on the small joint in the cervical spine known as the uncinate processes. This can cause the user to awaken with neck pain and muscle spasms and may even lead to osteoarthritis of these joints over time. Also, while lying supine, the shape of such prior art pillows often causes a forward protrusion of the head that can create an anterior weight bearing posture in the user, shortening the anterior cervical spine musculature and increasing the curve in the thoracic and lumbar spine, thereby resulting in hyper-kyphosis or hyper-lordosis. Such poor posture over time can lead to a variety of musculoskeletal problems with such symptoms as headaches, neck and/or back pain, numbness or tingling in arms or hands, etc. The poor spinal positioning and resulting poor posture caused by many prior art pillows can also irritate pre-existing problems such as arthritis and disc syndromes. Since the average person spends approximately one third of his or her life in a sleeping position and during sleep the human body repairs, it is important to be in a biomechanical position that will accommodate and enhance this process. The pillows that are the subject of the present invention fulfill these needs and yield further advantages, as described below, by generally providing an improved ergonomic design that promotes improved alignment of the neck and spine when a person is in a supine or sidelying position.

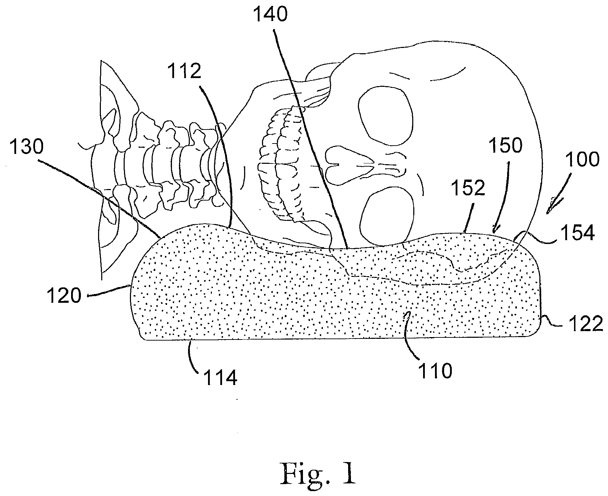

Figs. 1-5 illustrate a pillow 100 designed to promote improved alignment of the neck and spine when a person is in a sidelying position. As described below, the pillow 100 has a specific shape and contours to achieve this objective. The pillow 100 is formed of a viscoelastic material, such as a viscoelastic foam, which possess specific thermally responsive properties which cause the pillow 100 to conform to the shape of the portion of a person's body that contacts the pillow. The viscoelastic foam has a lower stiffness or hardness at an elevated temperature as compared to the stiffness at a cooler temperature. Conversely, conventional pillow filler materials typically have a constant stiffness with respect to a changing temperature. The body heat of the person acts to soften the portion of the pillow 100 in contact with the body, while the portion of the pillow 100 not contacting the body remains more firm. As a result, the pillow 100 allows for a greater comfort over a conventional pillow by accommodating each user's body form.

Any number of different viscoelastic foam materials are commercially available and can be selected for use in the present invention so long as they are suitable for the intended application and use as a pillow material. Generally, there are several important considerations when shopping for a memory foam product, such as the pillow of the present invention. Two of the main factors are the thickness and density. Memory foam comes in densities ranging from 11b to 51bs. Foam density of 5 pounds (lbs) per cubic foot (80 kg/m³) or greater is considered high quality, although most standard memory foam has a density of about 1 to 5 lb/ft³ (16-80 kg/m³). In addition, most bedding, such as topper pads and comfort layers in mattresses and pillows, has a foam density of between about 3 to 4.5 lb/ft³ (48-72 kg/m³). Very high densities such as 5.3 lb/ft³ (85 kg/m³) are used infrequently in mattresses. The pillows of the present invention can be formed of materials that have the above densities and in particular, can in one embodiment have densities between about 3 and 5 lbs (1.36 and 2.27 kg); between about 4 and 5 lbs (1.81 and 2.27 kg); between about 3 to 4.5 lbs (1.36 and 2.04 kg); between about 4 to 4.5 lbs (1.81 and 2.04 kg), etc.

For some bedding, memory foam typically comes in slab form from which the products, such as a mattress component, can be made and the slabs can have different thicknesses, typically in various thicknesses ranging from about 1 inch to about 5 inches (about 2.45 cm to about 13 cm). However, these are merely exemplary dimensions and properties and are not limiting of the present invention.

Viscoelastic foam products can be formed using any number of different processes including but not limited to pouring the liquid foam into a mold or the like to form a block which is then removed from the mold when cooled. More advanced technology creates the viscoelastic foam in a vacuum chamber. Called vacuum injection, this process of manufacturing creates a foam product of uniform density and ultimately, a high quality mattress topper, pad, or pillow. Various finishing techniques can be used to form the viscoelastic product having the desired shape and in particular, varies cutting techniques (e.g., a laser cut) can be used to transform the block of viscoelastic material into the end-use product.

The pillow 100 of Figs. 1-5 is formed of a body 110 (such as a viscoelastic foam body) that is defined by a top surface 112 on which the user's body rests; an opposite bottom surface 114, a first side 116, an opposite second side 118, a front 120 and an opposite rear 122. The pillow 100 has a length L1 and a width W1 and a variable thickness T1 across the width W1 of the pillow 100 as described below.

The pillow 100 is formed such that is has several distinct regions that are designed to contact and support the neck (spine) and head of the user. More specifically, the pillow 100 has a first region 130 formed along the front 120 of the pillow 100, a second region 140 formed centrally, and a third region 150 formed along the rear 122 of the pillow 100. The second region 140 is thus located between the first region 130 and the third region 150.

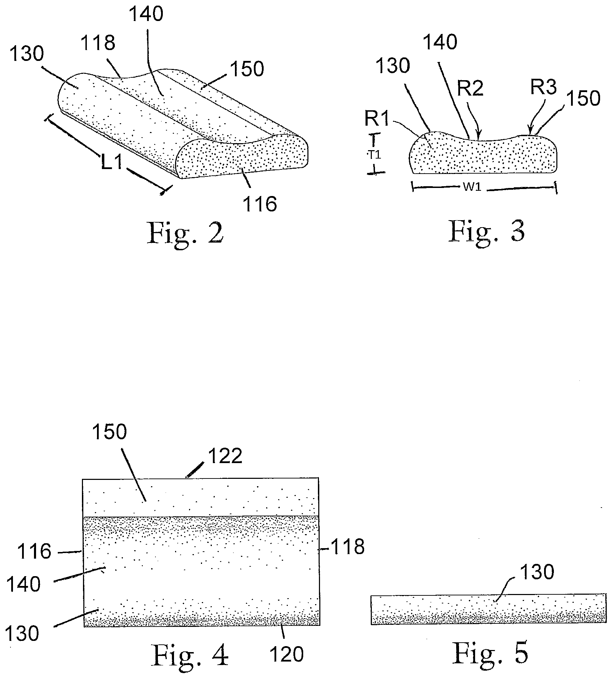

As can be seen from Figs. 1-5, the first region 130 and the third region 150 are both generally convex shaped portions, while the center second region 140 is concave in nature. As best shown in Fig. 3, the top surface of the pillow thus transitions from a convex surface of the first region 130 to a concave surface of the second region 140 before again transitioning to the convex surface of the third region 150. It will be appreciated that, as shown, the first region 130 has a more pronounced curvature compared to the third region 150 which is defined by a slight curvature. The curvature in the third region 150 can be so slight that a portion thereof has almost a flat appearance before the pillow has more pronounced curvature at the rear edge 122 of the pillow. In other words, the third region 150 can be defined by a complex curvature in that it can include a first section 152 that extends from a transition between the second region 140 and the third region 150 to a first point and a second section 154 that extends from the first point to the rear edge 122 of the pillow 100. The radius of curvature in the first section 152 is different than the radius of curvature in the second section 154 since as can be readily seen in Fig. 3, the curvature in the second section 154 is much more pronounced.

The convex first region 130 is defined by a first radius of curvature R1; the concave second region 140 is defined by a second radius of curvature R2; and similarly, the convex third region 150 is defined at least in part by a third radius of curvature R3. It will be appreciated that the transition points between the adjacent convex and concave regions can be defined by inflection points. In particular, the first region 130 which has a convex shape can be thought of as having a convex increasing (positive slope) section and a convex decreasing (negative slope) section and similarly, the second region can be thought of as having a concave increasing (positive slope) section and a concave decreasing (negative slope) section. The same is true of the third region 130 in that it can include a convex increasing (positive slope) section and a convex decreasing (negative slope) section.

The first region (roll portion) 130 is formed integral with the other regions such that its highest aspect is vertically higher than the top surface of the other two regions, namely, the second and third regions 140, 150, respectively. In other words, the thickness of the pillow 100 is greatest in the first region 130 (e.g., a roll portion) compared to both the second region 140 and the third region 150 and is at a minimum in the second region 140.

When the user is in a sidelying position as shown in Fig. 1, the neck 20 and head 30 of a user 10 are maintained in a substantially straight posture as supported by the first region (roll portion) 130 and the second and third regions 140, 150, respectively. More specifically, the neck 20 contacts and is supported by the first region 130 and this provides the proper alignment of the neck and spine.

The width W1 of the pillow 100 is constructed such that for many or most patients, the top of the head 30 extends to and at least partially seats in the first section 152 of the third region 150 when the user is in a sidelying position as shown in Fig. 1. However, it will be appreciated that a user's head 30 may be entirely contained within and in contact with the second region 140.

In one embodiment, the pillow has a length L1 of about 20.00 inches (about 508 mm); a width W1 of about 12.55 inches (about 319 mm); and a maximum thickness of about 3.57 inches (about 91 mm) as measured in the first region 130 (which is the thickest part of the pillow 100). The first region 130 can be defined by a first radius of curvature (R1) of 2.30 inches (58.4 mm); the second region 140 can be defined by a second radius of curvature (R2) of 9.12 inches (232 mm) and the third region 150 can be defined by a third radius of curvature (R3) of 6.69 inches (170 mm). As mentioned above, the third region 150 can be modified so as include a flat or a section of reduced curvature relative to adjacent sections. It will be appreciated that the above dimensions are merely exemplary in nature and do not limit the scope of the present invention.

It has been found that the construction of pillow 100 provides greater comfort and a therapeutic effect as a result of the curvature of the first region 130 for supporting the neck in an optimal manner resulting in a neutral spine position being obtained when the user is in the sidelying position.

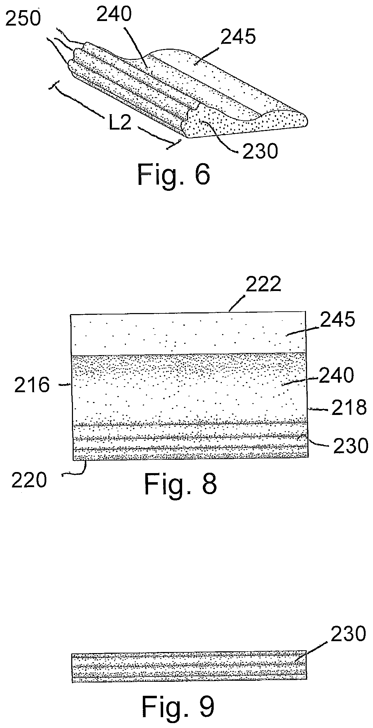

Now referring to Figs. 6-9 in which a pillow 200 according to an embodiment of the invention is shown. Pillow 200 is specifically constructed to promote improved alignment of the neck and spine when a person is in a supine position.

The pillow 200 of Figs. 6-9 is formed of a body 210 (such as a viscoelastic foam body) that is defined by a top surface 212 on which the user's body rests; an opposite bottom surface 214, a first side 216, an opposite second side 218, a front 220 and an opposite rear 222. The pillow 200 has a length L2 and a width W2 and a variable thickness T2 across the width W2 of the pillow 200 as described below.

The pillow 200 is formed such that is has several distinct regions that are designed to contact and support the neck (spine) and head of the user. More specifically, the pillow 200 has a first region 230 formed along the front 220 of the pillow 200, a second region 240 formed centrally, and a third region 245 formed along the rear 222 of the pillow 200. The second region 240 is thus located between the first region 230 and the third region 250.

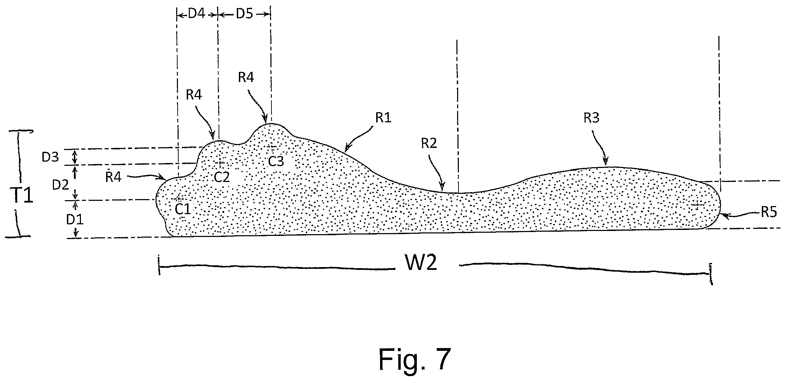

As can be seen from Figs. 6-9, the first region 230 and the third region 245 are both generally convex shaped portions, while the center second region 240 is concave in nature. As best shown in Fig. 7, the top surface of the pillow thus transitions from a convex surface of the first region 230 to a concave surface of the second region 240 before again transitioning to the convex surface of the third region 245. It will be appreciated that, as shown, the first region 230 has a more pronounced curvature compared to the third region 245 which is defined by a slight curvature.

Both the front 220 and rear 222 of the pillow are defined by rounded edges which enhances the comfort of the pillow 200.

While the first region 230 has a generally cylindrical shape (roll portion) it is further defined by a plurality of ribs 250 that are strategically placed along the convex shaped first region 230. In particular, the first region 230 can be thought of as being defined by a first section that extends from the front 220 to an apex (maximum) point at which point the slope of the curve changes. A second section of the first region 230 extends from the apex point to a transition (inflection) point that marks the beginning of the concave shaped second region 240. In other words and as shown, the first section can be thought as being an upwardly sloped section (convex increasing) of the convex shaped first region 230, while the second section can be thought of as being a downwardly sloped section (convex decreasing) of the convex shaped first region 230.

As shown in Fig. 7, there are three ribs 250 that are located along the first region 230 and in particular, are located along the first section (convex increasing section) thereof. According to the invention, the ribs 250 are entirely contained within the first section and are not disposed within the second section. In other words, the ribs 250 are located entirely along the upwardly sloped portion (convex increasing) of the convex shaped first region 230 and are not present and located along the second section. The present applicant has found, as discussed in greater detail below, that the position of spaced apart ribs 250 on the upwardly slope portion of the convex region (roll portion) 230, with the downwardly sloped portion thereof being free of ribs results in a comfortable pillow that is also therapeutic in that the strategic positioning of the ribs 250 promotes improved alignment of the neck and spine for reasons discussed below.

Cervical lordosis is a curve in the cervical spine, the area of the spine which contains the neck vertebrae. This curve is entirely normal and in fact desirable because it helps to stabilize the head and spine, but when the curve straightens out, becomes too deep, or faces in the wrong direction, it can become a problem. There are several treatments available for loss of cervical lordosis, with treatment being supervised by a medical professional who specializes in spinal care.

The normal cervical lordosis (which extends from C1 to T2) should have a 17-24 cm. radius, based on the patient's height. This is easily measured with the AcuArc ruler which is one of the commonly used techniques to measure the cervical curve of the spine.

In an optimal cervical lordosis condition, all segments should be on Georges's line (posterior body line) which is a curved line that should touch the posterior body margin of all of the segments of the spin in any of the three main curvatures. There should be an even spacing between each spinous process. Positioning of the head and spine should also be assessed for anterior head placement (also known as Forward Head Posture). The normal cervical lordosis (which extends from C1 to T2) should have a 17-24 cm. radius, based on the patient's height. This is easily measured with the AcuArc ruler. The posterior arch of Atlas should be centered in the space between occiput and the C2 spinous process. If C1's posterior arch "crowds" occiput, it is labelled as an "inferior" Atlas. If it crowds C2, it is labelled "superior". The normal Atlas Plane line would be 18-24 degrees superior to the bottom of the film. A line under the bottom of the C2 body (Whitehorn's line) should be parallel with the floor.

The structure of the pillow 200 allows the head 30 to arch back over the cylindrical shaped roll portion (first region 230) and be supported by the center second section (second region 240) of the pillow 200.

Each of the ribs 250 is also a curved structure and as illustrated, each rib 250 has a convex shape. The ribs 250 are spaced from one another such that the regions between two adjacent ribs 250 represent a valley that has a concave curvature. The ribs 250 can thus be defined by a radius of curvature (R4). The ribs 250 can be formed, as shown, such that they are uniform in that the radius of curvature (R4) of each of the ribs 250 is at least substantially equal.

In one embodiment, the first region 230 is defined by a first radius of curvature (R1) having a value of about 3.944 inches (about 100 mm) and each of the ribs 250 has a fourth radius of curvature (R4) of about 0.500 inch (about 12.7 mm). However these values are merely exemplary in nature and not limiting of the present invention and other values can be selected and used. The concave second region 240 is defined by a second radius of curvature (R2) having a value of about 3.927 inches (about 99.75 mm). One will thus appreciate that the values of the radiuses of curvature of the first and second regions 230 and 240 are very similar with the second region 240 only being slightly less. This is in contrast to the third region 245 which does not have as pronounced a curvature and in the illustrated embodiment is defined at least in part by a third radius of curvature (R3) of 6.693 inches (170 mm). The rear 222 can be defined by a fifth radius of curvature (R5) which can be about 0.500 inch (about 12.7 mm) in one embodiment. The curvature R1 is much more pronounced than R3.

As shown in Fig. 7, the spacing between the ribs 250 does not have to be uniform in that the distance between the center points (C1, C2, C3) of the circles that define the radii of curvature of the ribs 250 can be different between the forward rib 251 (associated with center point C1) and a center rib 253 (associated with center point C2) as compared to the center rib 253 and a rear rib 255 (associated with center point C3). For example, the distance (D4) between the forward rib 251 and the center rib 253 can be about 0.917 inch (about 23.3 mm), while the distance (D5) from the center rib 253 to the rear rib 255 can be about 1.147 inch (about 29.1 mm) (as measured along the width of the pillow (i.e., front to rear measurement)). The center points C1, C2, C3 are thus at different heights relative to a ground surface on which the bottom 214 of the pillow seats. In the illustrated embodiment, the distance (D1) from point C1 to the ground surface is about 0.795 inch, while the distance (D2) from point C2 to the ground surface is about 1.586 inch, i.e. about 40.3mm (distance between C1 and C2 being about 0.791 inch, i.e. about 20.1 mm) and the distance (D3) from point C3 to the ground surface is about 1.941 inch, i.e. about 49.3 mm (distance between C2 and C3 being about 0.355, or 9 mm).

In the illustrated embodiment, the length L2 is about 20.00 inches (about 508 mm), the width W2 is about 12.50 inches (about 317.5 mm) and the maximum thickness T2 is about 2.44 inches (about 62.0 mm) (as defined in the first region 230). Once again, the pillow 200 can have any number of other dimensions depending upon the particular application and intended use.

It will be appreciated that the dimensions of the embodiment shown in Fig. 7 are merely exemplary in nature and not limiting of the present invention.

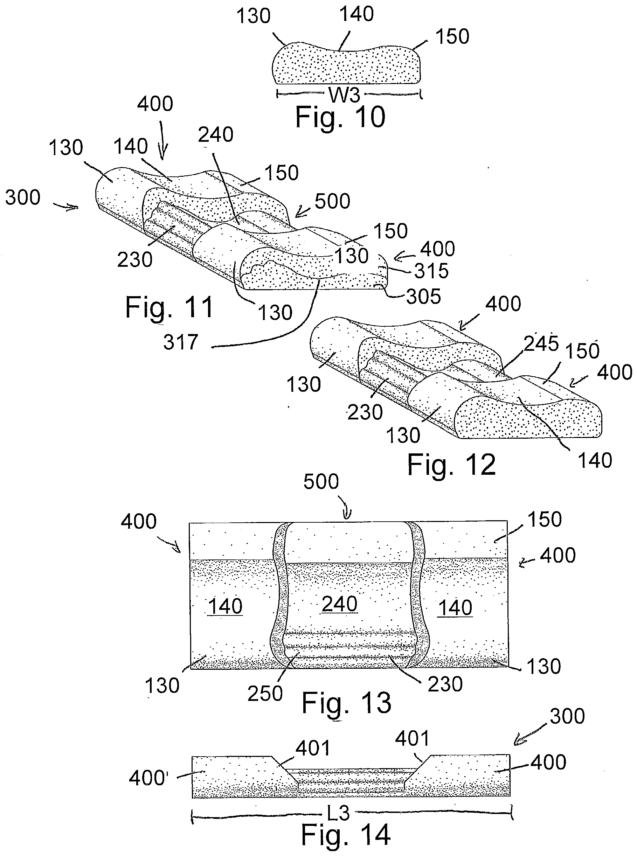

In Figs. 10-15, a third pillow 300 is shown and is specifically constructed to be a hybrid/multifunctional pillow in that the construction of the pillow 300 is designed to accommodate both persons that sleep in the supine position and in the sidelying position. For example, many people do not maintain the same sleep position all night and instead move between different positions. Amongst this group of people it is most common that people move between the supine position and the sidelying position. As a result, it is desirable for a pillow to accommodate both positions and offer a therapeutic benefit in both positions. The pillow 300 achieves this objective.

The pillow 300 thus shares similarities to both the pillow 100 (sidelying position pillow) and the pillow 200 (supine position pillow). In particular, the pillow 300 includes at least one first section 400 that has a construction that is identical or very similar to the construction of the pillow 100 and has at least one second section 500 that has a construction that is identical or very similar to the construction of the pillow 200. In the illustrated embodiment, the pillow 300 includes a single second region 500 and includes two first sections 400, with the second section 500 being formed between the two first section 400 and therefore, the first sections 400 define the two ends (lateral sides) of the pillow 300. As a result, in the above arrangement, a supine sleeping position is in the center of the pillow, while the sidelying positions are adjacent thereto and are located on either side of the supine sleeping position. The user thus can roll in either the left direction or the right direction from the center supine position to one of the respective sidelying positions and conversely, can move from one of the sidelying position to the center supine position.

It will be appreciated that the pillow 300 can be formed such that it only consists of a single first section 400 and a single second section 500 located side-by-side.

In the illustrated embodiment, the construction of the different sections 400, 500 of the pillow 300 can be the same as the constructions of the individual pillows 100, 200 and therefore, like elements are numbered alike. In other words, the first section 400 includes the first region 130, second region 140 and third region 150 having the contours described hereinbefore with reference to pillow 100. Similarly, the second section 500 includes the first region 230, second region 240, and third region 245 and thus, has ribs 250 as described herein in the first region 230.

As shown in Figs. 11-15, the second section 500 has a thickness that is less than the first section(s) 400 and is thus recessed relative to the other adjacent section(s) 400.

It will be appreciated that the relative dimensions, such as the width, of each pillow section can be varied depending upon the particular application, such as the pillow size (e.g., standard, queen, king, etc.).

More specifically, since the pillow 300 is a hybrid pillow and includes both supine and sidelying sections, some of the dimensions of the pillow 300 are increased. For example, the length (L3) of the pillow 300 can be about 27.00 inches (about 68.6 cm), while the width (W3) is about 12.5 inches (about 317.5 mm) (as with the other pillows 100, 200). Each first section 400 can have a length of about 9.00 inches (about 229 mm) and therefore, when there are two first sections 400 as shown in Fig. 11-15, the single center second section 500 likewise has a length of about 9.00 inches (about 229 mm). Thus, while in the illustrated embodiment, the three sections 400, 500, 400 have the same length, the pillow 300 can made such that the sections are of different lengths. For example, the second section 500 can have a length that is greater than the length of the one or more first sections 400 and alternatively, the second section 500 can have a length that is less than the sections 400.

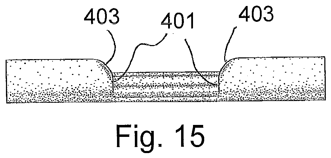

The transition between the first section 400 and the second section 500 can have any number of different structures, including those shown in Figs. 14 and 15. In particular, in Fig. 14, the inner edges 401 of the first sections 400 can be in the form of beveled edges, such as a beveled edge that is formed at a 45 degree angle as shown. This beveled edge 401 provides a ramp like structure between the section 400 and section 500 that permits the user to move from one region to the other region and vice versa. The edge 401 can be formed at other angles as well.

In Fig. 15, the edge 401 is formed at a substantially 90 degree angle; however, the edge 401 includes a rounded top 403. This provides a softened edge that allows the user to more easily move from one section 400, 500 to the other section.

In addition, while the two free ends of the pillows illustrated in Figs. 1-15_have a 90 degree clean cut edge, it will be appreciated that the ends of the pillow can be formed to have other shapes such as a more rounded shape or another shape.

The pillow 300 can be formed using any number of conventional techniques, such as those described hereinbefore. In one embodiment, the pillow 300 can be formed as a single piece construction in that the different regions 400, 500 of the pillow 300 are formed in a common manufacturing process, such as a common mold process (vacuum injection mold process). As a result, the pillow 300 has a single unitary, integral construction.

Alternatively as shown in Fig. 11, the pillow 300 can be formed as a two piece construction in that the pillow 300 can include an underlying base layer 305 and a top layer 315 that mates with the base layer 305 to form the pillow 300 with the regions 400, 500. For example, the base layer 305 and top layer 315 can be formed in separate molding operations to form the separate contoured pillow base layer 305 and top layer 315. It will be appreciated that the base layer 305 has the form of the pillow 200 (supine position pillow) and the top layer 315 has the form of the pillow 100 at least in part. In particular, a top surface of the top layer 315 has the contoured shape of the pillow 100 in that the top surface of the top layer 315 includes the regions 130, 140, 150 that provide the therapeutic effect for when a user is in a sidelying position.

An underside 317 of the top layer 315 is thus a mirror image of the top surface of the base layer 305 (pillow 200) such that when the two mate together with the underside 317 mating with the top surface of the base layer 305, a clean fit results. The two parts 305, 315 can be adhered to one another using conventional techniques including but not limited to using an adhesive that is placed between the two parts 305, 315.

Any one of the pillows described and illustrated herein can include a locator member that easily permits the user to determine the positioning of the pillow within a pillowcase. For example, the pillow can have a tab (protrusion) that extends outwardly from one section of the pillow, such as at a front edge in order to easily to provide a tactile indicator to a user to allow the position of the pillow to be easily determined especially when the pillow may be covered with a pillowcase or the like.

In one embodiment, all of the pillows described herein are constructed to fit within a standard pillowcase as opposed to requiring a custom pillowcase. Alternatively, the pillow can be fitted with a custom pillowcase that is cut to the shape of the underlying pillow.

It will also be understood that an accessory (not shown) can be provided for use with the pillow 300 in that a middle top section that is similar to or identical to the part 315 can be provided for insertion into the open space above the section 500 between the two sections 400.

The underside surface of the accessory thus has a shape that is a mirror image of the top surface of the section 500 to allow a clean, intimate mating (flush fit) between the accessory and the second section 500. The accessory can be used to transform the pillow 300 into a complete sideyling pillow. In addition, the accessory can have different material characteristics compared to the parts 315 that formed the sections 400. For example, the density (e.g., foam density) can be different than the parts 315 to provide a different feel in the center section compared to the other sides. The accessory can be freely removed from the pillow 300. One or more fasteners, such as hook and loop material can be provided in one or more areas that are not in contact with the user of the pillow 300 for providing some means for securing the accessory to the pillow 300.

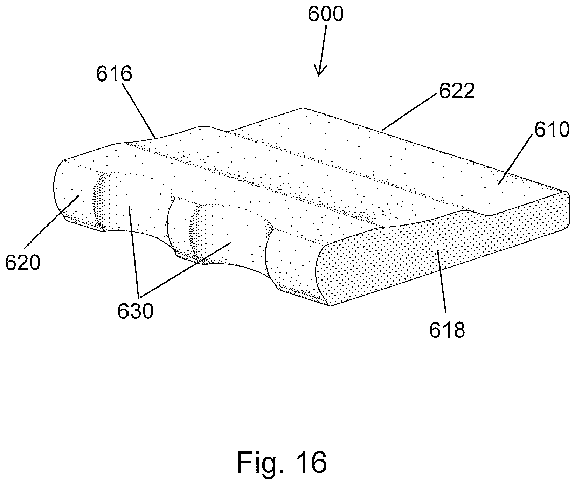

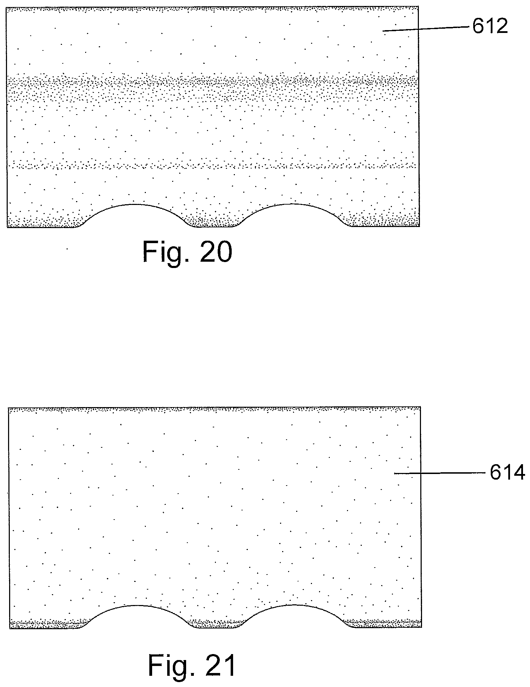

Now referring to Figs. 16-21, a pillow 600 is formed of a body 610 (such as a viscoelastic foam body) that is defined by a top surface 612 on which the user's body rests; an opposite bottom surface 614, a first side 616, an opposite second side 618, a front 620 and an opposite rear 622. The pillow 600 has a length L1 and a width W1 and a variable thickness T1 across the width W1 of the pillow 600 as described below.

The pillow 600 is of a type that is configured for use with side sleeping individuals.

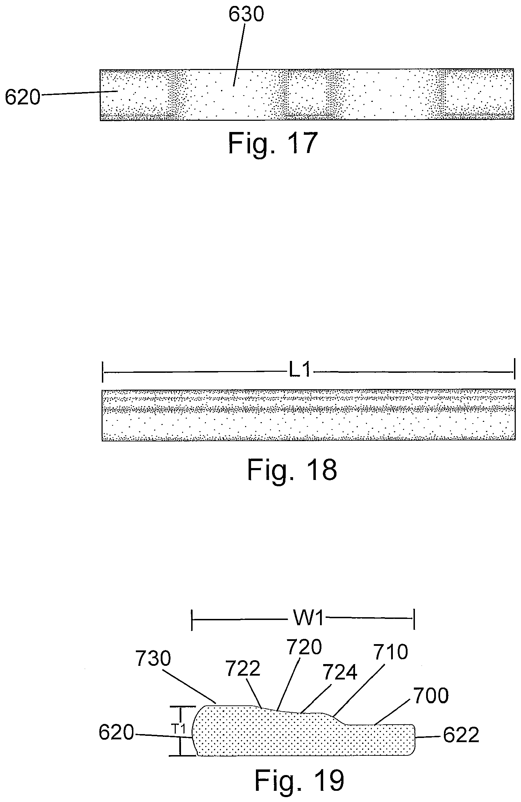

The pillow 600 is formed such that is has several distinct regions that are designed to contact and support the neck (spine) and head of the user. As best shown in the side views of Figs. 16 and 19, the pillow 600 has a number of different regions from the front edge 620 to the rear edge 622. From the front edge 620 to the rear edge 622, the pillow 600 has a step construction with the pillow 600 having a maximum thickness at the front edge 620 and a minimum thickness at the rear edge 622. Between these two areas, the thickness of the pillow 600 varies depending upon the particular regions of the pillow 600 and has a step-like construction.

The front edge 620 includes a pair of cut-outs 630 that are each designed to fit a shoulder of an individual. Each cut-out 630 is in the formed pillow body and has a curved shape and in particular, has an arcuate shape (e.g., crescent shape). The depth of the cut-out 630 is about 1.50 inches (38.1 mm) as measured from the front edge 620 to a center point of the cut-out 630. The two cut-outs 630 can have equal lengths (7.81 inches, 198 mm) are spaced apart from one another a prescribed distance (e.g., 1.98 inches, 50.3 mm) that is selected such that if a person's shoulder (such as the left shoulder) is disposed within one cut-out 630 (such as the right cut-out 630) and then the user rolls over for sleeping on the opposite side, the user's other shoulder (e.g., right shoulder) will naturally fall into the other cut-out 630 (i.e., left cut-out 630). It will be appreciated that the formation of two cut-outs 630 in the pillow 600 prolongs the life of the pillow 600 since the pillow 600 offers two distinct pillow regions that can accommodate a user's shoulder. Thus, the user does not have to use the same region of the pillow every night but instead can prolong life of the pillow 600 by periodically alternating the pillow region that is used. The shoulder cut-outs 630 thus serve as a locating feature for locating the shoulder of the user in a side sleeping position.

As described herein, the various regions of the pillow 600 are specifically designed to provide equal pressure across the head and neck areas of the individual as the individual rests his or head on the pillow 600 in the manner described herein. The pillow 600 can have a length of about 26.0 inches (about 66 cm) and a width of about 14.10 inches (about 35.8 cm).

The pillow 600 includes a first region 700 that terminates at the rear edge 622 and includes the cut-outs 630. The first region 700 is a planar region in which the thickness of the pillow 600 is uniform. A second region 710 is a region of the pillow 700 that has incline (upward slope) in the direction from the rear edge 622 to the front edge 620. The second region 710 thus has variable thickness.

The pillow 600 includes a third region 720 that is adjacent the second region 710. The third region 720 is a region in which the thickness of the pillow does vary in that there is a slight increase in the forward direction of the pillow 600. The third region 720 has a slight concave shape. The third region 720 can have a variable slope in itself in that a front portion 722 of the third region 720 has a greater slope than a rear portion 724 of the third region 720. The front portion 722 represents the second step of the pillow 600. The slope of this second step is less than the slope of the first step defined by the second region 720.

However, the slope of the second region 710 is substantially greater than the slope of the third region 720 since the second region 710 serves as a distinct "step" construction between two relatively planar sections.

The pillow also includes a fourth region 730 that defines the front edge 620 of the pillow 600. The fourth region 730 is a planar section in that the thickness of the fourth region 730 is at least substantially uniform along the fourth region 730. The first and fourth regions can thus be planar sections that lie in different planes (i.e., parallel planes).

As shown, the front edge 620 can be a curved edge (e.g., rounded edge (convex edge)), while the rear edge 622 can be a substantially flat edge.

As previously mentioned, the pillow 600 has a construction that provides for equal pressure across the neck and head of the user and more particularly, the stepped construction allows for compression of the pillow such that the spine is maintained in a nuetral position. When a user rests his or her head on the pillow 600, the top of the head will seat on and be supported by the first region 700. The user's head extends along the first region 700, the second region 710 and at least the rear portion 724 of the third region 720. The neck of the user rests on the fourth region 730 and at least partially on the third region 720 (e.g., the front portion 722 thereof).

The fourth region 730 has increased thickness compared to the other regions and thus represents a higher structure since in a side sleeping position, the natural shape of the neck includes an inward taper since the head has a greater width than the neck.

The stepped construction provides different thicknesses of material and therefore, has a complex compression profile across the width of the pillow 600. However, the compression profile is designed such that when the pillow 600 is in use, the spine is maintained in a substantially neutral position. In other words, the stepped construction of the pillow 600 is configured such that the head and neck (spin) are supported evenly across the width of the pillow 600. In other words, the different compression profiles of the different regions of the pillow 600 based on the thickness of the pillow 600 in the region and based on the contour of the pillow in the region cause the pillow to compress so as to maintain the user's spine in the neutral position. The pillow 600 includes a maximum thickness of about 3.25 inches (82.6 mm).

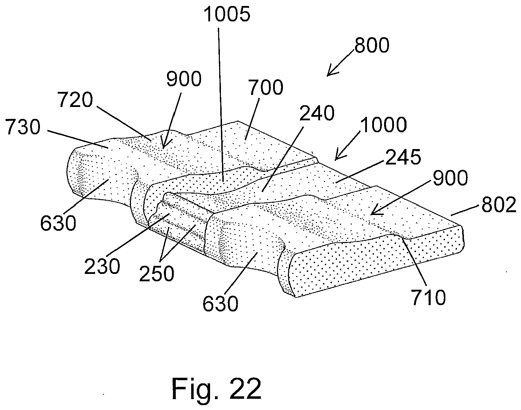

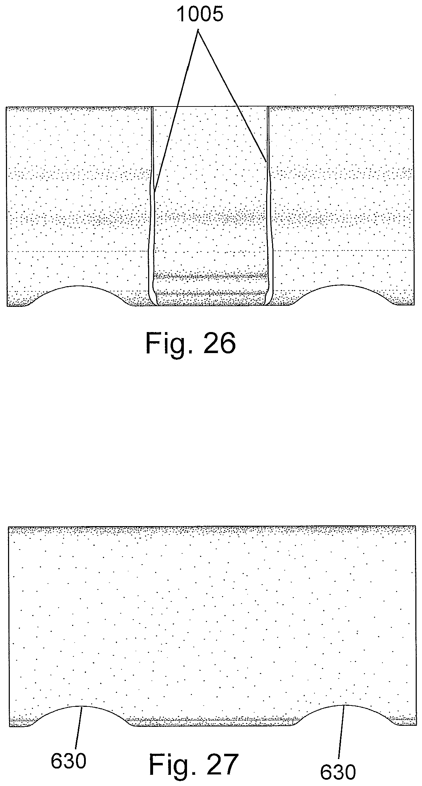

In Figs. 22-27, a pillow 800 is shown and is specifically constructed to be a hybrid/multifunctional pillow in that the construction of the pillow 800 is designed to accommodate both persons that sleep in the supine position and in the sidelying position. For example, many people do not maintain the same sleep position all night and instead move between different positions. Amongst this group of people it is most common that people move between the supine position and the sidelying position. As a result, it is desirable for a pillow to accommodate both positions and offer a therapeutic benefit in both positions. The pillow 800 shares some similarity to pillow 300 described herein.

The pillow 800 thus shares similarities to both the pillow 600 (sidelying position pillow) and the pillow 200 (supine position pillow). In particular, the pillow 800 includes at least one first section 900 that has a construction that is identical or very similar to the construction of the pillow 600 and has at least one second section 1000 that has a construction that is identical or very similar to the construction of the pillow 200. In the illustrated embodiment, the pillow 800 includes a single second region 1000 and includes two first sections 900, with the second section 1000 being formed between the two first sections 900 and therefore, the first sections 900 define the two ends (lateral sides) of the pillow 800. As a result, in the above arrangement, a supine sleeping position is in the center of the pillow, while the sidelying positions are adjacent thereto and are located on either side of the supine sleeping position. The user thus can roll in either the left direction or the right direction from the center supine position to one of the respective sidelying positions and conversely, can move from one of the sidelying position to the center supine position.

It will be appreciated that the pillow 800 can be formed such that it only consists of a single first section 900 and a single second section 1000 located side-by-side.

In the illustrated embodiment, the construction of the different sections 900, 1000 of the pillow 800 can be the same as the constructions of the individual pillows 600, 200 and therefore, like elements are numbered alike. In other words, the first section 900 includes the regions 700-730 having the contours described hereinbefore with reference to pillow 600. Similarly, the second section 1000 includes the first region 230, second region 240, and third region 245 and thus, has ribs 250 as described herein in the first region 230.

As shown in Figs. 22-24 and 26, the second section 1000 has a thickness that is less than the first section(s) 900 and is thus recessed relative to the other adjacent section(s) 900. As a result, a side wall 1005 is located between the first section 900 and the second section 1000. The side wall 1005 is thus a transition wall between the two sections and as shown in the figures, the side wall 1005 has a variable height in that at a rear 802 of the pillow 800, the side wall 1005 has a minimum height. The side wall 1005 can also be formed at an angle (beveled wall) as shown in the figures.

It will be appreciated that the relative dimensions, such as the width, of each pillow section can be varied depending upon the particular application, such as the pillow size (e.g., standard, queen, king, etc.).

More specifically, since the pillow 800 is a hybrid pillow and includes both supine and sidelying sections, some of the dimensions of the pillow 800 can be increased. For example, the length of the pillow 800 can be about 26.00-27.00 inches (about 66-68.6 cm), while the width is about 14.1 inches (about 35.8 cm) (as with the other pillows 100, 200). In one illustrated embodiment, the three sections 900, 1000, 900 can have the same length, the pillow 800 can made such that the sections are of different lengths. For example, the second section 1000 can have a length that is greater than the length of the one or more first sections 900 and alternatively, the second section 1000 can have a length that is less than the sections 900.

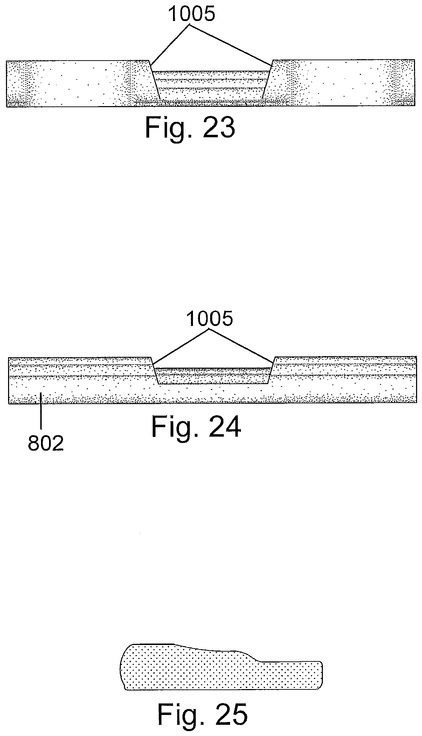

The transition between the first section 900 and the second section 1000 can have any number of different structures, including those shown in Figs. 23-24. In particular, in Figs. 23-24, the inner edges of the first sections 900 can be in the form of beveled edges, such as a beveled edge as shown. This beveled edge provides a ramp like structure between the section 900 and section 1000 that permits the user to move from one region to the other region and vice versa.

The inner edge can be formed at a substantially 90 degree angle and can include a rounded top. This provides a softened edge that allows the user to more easily move from one section 900, 1000 to the other section.

In addition, while the two free ends of the pillows illustrated in Figs. 1-27 have a 90 degree clean cut edge, it will be appreciated that the ends of the pillow can be formed to have other shapes such as a more rounded shape or another shape.

The pillow 800 can be formed using any number of conventional techniques, such as those described hereinbefore. In one embodiment, the pillow 800 can be formed as a single piece construction in that the different regions 900, 1000 of the pillow 800 are formed in a common manufacturing process, such as a common mold process (vacuum injection mold process). As a result, the pillow 800 has a single unitary, integral construction. Alternatively, the pillow 800 can be formed as a two piece construction in that the pillow 800 can include an underlying base layer and a top layer that mates with the base layer to form the pillow 800 with the regions 900, 1000. For example, the base layer and top layer can be formed in separate molding operations to form the separate contoured pillow base layer and top layer.

The invention is described in detail with reference to particular embodiments thereof, but the scope of the invention is to be gauged by the claims that follow. Reference signs have been included in the claims to increase their intelligibility. The reference signs are not intended to limit the scope of the claims.

## Claims

1. A pillow (200, 800) comprising:
a foam body (210) having a top surface (212), an opposing bottom surface (214), a rear edge (222) and an opposing front edge (220) and lateral sides (118, 218), wherein the foam body is defined by a first region (230) that includes the front edge, a second region (240) adjacent the first region and a third region (245) adjacent the second region and defining the rear edge, wherein the first region has a convex shape, the second region has a concave shape and the third region has a convex shape, the pillow having a maximum thickness in the first region and a minimum thickness in the second region, the first region having a first section that extends to the front edge and has a positive slope and an adjacent second section that has a negative slope; **characterized by**: three ribs (250) extending longitudinally between the lateral sides and being located exclusively within the first section of the first region, the second section of the first region being free of ribs; the three ribs (25) comprising a front rib (251), a middle rib (253) and a rear rib (255), with a first distance (D4) being defined between the front rib and middle rib and a second distance (D5) being defined between the middle rib and the rear rib, as measured along a width (W2) of the pillow and optionally wherein the three ribs have convex shapes with a radius of curvature (R4) that defines each rib being at least substantially equal; and
wherein the front rib (251) defines a forwardmost point of the foam body (210).

2. The pillow (800) of claim 1, for use in both a supine sleeping position and a sidelying sleeping position:
wherein the foam body is defined by a first section (1000) constructed for use when a user is in the supine sleeping position and a pair of adjacent second sections (900) for use when the user in the sidelying position, the first section being disposed between the second sections;
wherein the first section (1000) of the foam body includes the said first, second and third regions (230, 240, 245) and the three ribs (250); and
wherein the adjacent second sections of the foam body includes a pair of arcuate shaped cut-outs (630) formed in the front edge, the arcuate-shaped cut-outs being spaced apart along the front edge for receiving a shoulder of a user in the sidelying position.

3. The pillow (200, 800) of claim 1 or 2, wherein the foam body (210) is formed of viscoelastic foam, whereby a head and neck of a user arches back over the first region (230) as supported by the foam body when the user is in a supine position with the head being at least substantially contained in the second region (240).

4. The pillow (200, 800) of claim 1, wherein the ribs (250) extend along the entire length (L2) of the foam body (210) from one lateral side (216) to the other lateral side (218).

5. The pillow (200, 800) of claim 1, wherein the first region (230) is defined by a first radius of curvature (R1) and the second region (240) is defined by a second radius of curvature (R2), and the third region (245) is defined by a third radius of curvature (R3), the third radius of curvature being greater than the first radius of curvature.

6. The pillow (200, 800) of claim 5, wherein the first radius of curvature (R1) is about 3.944 inches (about 100 mm); the second radius of curvature (R2) is about 3.927 inches (about 99.75 mm) and the third radius of curvature (R3) is about 6.693 inches (about 170 mm).

7. The pillow (800) of claim 2, wherein the second section (900) has a step configuration.

8. The pillow (800) of claim 7, wherein the second section includes a pair of steps (710, 722) located between first and second planar portions that are located at the front edge and rear edge, respectively, of the pillow, and optionally wherein the first and second planar portions are parallel to one another.

9. The pillow (800) of claim 8, wherein the pair of steps includes a first step (710) having a first slope and a second step (722) having a second slope, the first slope being greater than the second slope and located closer to the rear edge, optionally wherein a length of the second step is greater than a length of the first step.

10. The pillow (800) of claim 2, wherein an inner beveled wall (1005) is formed at an interface between the first section (1000) and each second section (900), the inner beveled wall having a varying height as measured from the rear edge to the front edge.

## Patentansprüche

1. Kissen (200, 800), Folgendes beinhaltend:
einen Schaumkörper (210), besitzend eine Oberseite (212), eine gegenüberliegende Unterseite (214), eine hintere Kante (222) und eine gegenüberliegende Vorderkante (220) und seitliche Seiten (118, 218), wobei der Schaumkörper durch einen ersten Bereich (230) definiert wird, beinhaltend die Vorderkante, einen zweiten Bereich (240), angrenzend an den ersten Bereich, und einen dritten Bereich (245), angrenzend an den zweiten Bereich und die hintere Kante definierend, wobei der erste Bereich eine konvexe Form besitzt, der zweite Bereich eine konkave Form besitzt und der dritte Bereich eine konvexe Form besitzt, wobei das Kissen eine maximale Dicke im ersten Bereich und eine minimale Dicke im zweiten Bereich besitzt, wobei der erste Bereich einen ersten Abschnitt besitzt, welcher sich zur Vorderkante erstreckt und eine positive Neigung besitzt, und einen angrenzenden zweiten Abschnitt, der eine negative Neigung besitzt; **gekennzeichnet durch**:
drei Rippen (250), welche sich in Längsrichtung zwischen den seitlichen Seiten erstrecken und ausschließlich innerhalb des ersten Abschnitts des ersten Bereichs befindlich sind, wobei der zweite Abschnitt des ersten Bereichs keine Rippen aufweist;
wobei die drei Rippen (25) eine vordere Rippe (251), eine mittlere Rippe (253) und eine hintere Rippe (255) beinhalten, wobei ein erster Abstand (D4) zwischen der vorderen Rippe und der mittleren Rippe definiert wird und ein zweiter Abstand (D5) zwischen der mittleren Rippe und der hinteren Rippe definiert wird, gemessen entlang einer Breite (W2) des Kissens, und wahlweise wobei die drei Rippen konvexe Formen mit einem Krümmungsradius (R4) besitzen, der jede Rippe dergestalt definiert, das sie zumindest im Wesentlichen gleich ist; und
wobei die vordere Rippe (251) einen vordersten Punkt des Schaumkörpers (210) definiert.

2. Kissen (800) nach Anspruch 1, zum Gebrauch gleichermaßen in einer auf dem Rücken liegenden Schlafposition und in einer auf der Seite liegenden Schlafposition:
wobei der Schaumkörper durch einen ersten Abschnitt (1000) definiert wird, konstruiert zum Gebrauch, wenn ein Benutzer sich in der auf dem Rücken liegenden Schlafposition befindet, und ein Paar von angrenzenden zweiten Abschnitten (900), zum Gebrauch, wenn der Benutzer sich in der auf der Seite liegenden Schlafposition befindet, wobei der erste Abschnitt zwischen den zweiten Abschnitten angeordnet ist;
wobei der erste Abschnitt (1000) des Schaumkörpers die ersten, zweiten und dritten Bereiche (230, 240, 245) und die drei Rippen (250) beinhaltet; und
wobei die angrenzenden zweiten Abschnitte des Schaumkörpers ein Paar von borgenförmig geformten Ausschnitten (630) beinhalten, welche in der vorderen Kante gebildet sind, wobei die borgenförmig geformten Ausschnitte entlang der vorderen Kante zur Aufnahme einer Schulter eines Benutzers in der auf der Seite liegenden Schlafposition voneinander entfernt angeordnet sind.

3. Kissen (200, 800) nach Anspruch 1 oder 2, wobei der Schaumkörper (210) aus viskoelastischem Schaum gebildet ist, wobei sich ein Kopf und ein Hals eines Benutzers rückwärts über den ersten Bereich (230) wölben, hierbei durch den Schaumkörper gestützt, wenn der Benutzer sich in einer auf dem Rücken liegenden Schlafposition befindet, wobei der Kopf mindestens im Wesentlichen in dem zweiten Bereich (240) bleibt.

4. Kissen (200, 800) nach Anspruch 1, bei welchem die Rippen (250) sich über die gesamte Länge (L2) des Schaumkörpers (210) von einer seitlichen Seite (216) bis zur anderen seitlichen Seite (218) erstrecken.

5. Kissen (200, 800) nach Anspruch 1, bei welchem der erste Bereich (230) durch einen ersten Krümmungsradius (R1) definiert wird und der zweite Bereich (240) durch einen zweiten Krümmungsradius (R2) definiert wird und der dritte Bereich (245) durch einen dritten Krümmungsradius (R3) definiert wird, wobei der dritte Krümmungsradius größer ist als der erste Krümmungsradius.

6. Kissen (200, 800) nach Anspruch 5, bei welchem der erste Krümmungsradius (R1) ungefähr 3,944 Zoll (ungefähr 100 mm) beträgt, der zweite Krümmungsradius (R2) ungefähr 3,927 Zoll (ungefähr 99,75 mm) und der dritte Krümmungsradius (R3) ungefähr 6,693 Zoll (ungefähr 170 mm) beträgt.

7. Kissen (800) nach Anspruch 2, bei welchem der zweite Abschnitt (900) eine Stufenkonfiguration besitzt.

8. Kissen (800) nach Anspruch 7, bei welchem der zweite Abschnitt ein Paar Stufen (710, 722) beinhaltet, welches zwischen einem ersten und einem zweiten planaren Abschnitt befindlich ist, welche jeweils an der vorderen Kante und der hinteren Kante des Kissens befindlich sind, und wahlweise bei welchem der erste und der zweite planare Abschnitt parallel zueinander sind.

9. Kissen (800) nach Anspruch 8, bei welchem ein Paar von Stufen eine erste Stufe (710) beinhaltet, welche eine erste Neigung besitzt, und eine zweite Stufe (722), welche eine zweite Neigung besitzt, wobei die erste Neigung größer ist als die zweite Neigung und näher an der hinteren Kante befindlich ist, wahlweise wobei eine Länge der zweiten Stufe größer ist als eine Länge der ersten Stufe.

10. Kissen (800) nach Anspruch 2, bei welchem eine innere an innere, abgeschrägte Wand (1005) an einer Schnittstelle zwischen dem ersten Abschnitt (1000) und jedem zweiten Abschnitt (900) gebildet ist, wobei die innere, abgeschrägte Wand eine variable Höhe, gemessen von der hinteren Kante bis zur vorderen Kante, besitzt.

## Revendications

1. Oreiller (200, 800), comprenant :
un corps en mousse (210) comportant une surface supérieure (212), une surface inférieure opposée (214), un bord arrière (222) et un bord avant opposé (220), ainsi que des côtés latéraux (118, 218), dans lequel le corps en mousse est défini par une première région (230) incluant le bord avant, une deuxième région (240) adjacente à la première région, et une troisième région (245) adjacente à la deuxième région et définissant le bord arrière, dans lequel la première région a une forme convexe, la deuxième région a une forme concave et la troisième région a une forme convexe, l'oreiller ayant une épaisseur maximale dans la première région et une épaisseur minimale dans la deuxième région, la première région comportant une première section s'étendant vers le bord avant et présentant une pente positive, et une deuxième section adjacente présentant une pente négative ; **caractérisé par** :
trois nervures (250) s'étendant longitudinalement entre les côtés latéraux et agencées exclusivement dans la première section de la première région, la deuxième section de la première région étant exempte de nervures ;
les trois nervures (25) comprenant une nervure avant (251), une nervure centrale (253) et une nervure arrière (255), une première distance (D4) étant définie entre la nervure avant et la nervure centrale, et une deuxième distance (D5) étant définie entre la nervure centrale et la nervure arrière, mesurée le long d'une largeur (W2) de l'oreiller, et dans lequel les trois nervures ont optionnellement des formes convexes, un rayon de courbure (R4) définissant chaque nervure étant au moins sensiblement égal ; et
dans lequel la nervure avant (251) définit un point le plus vers l'avant du corps en mousse (210).

2. Oreiller (800) selon la revendication 1, destiné à être utilisé dans une position couchée sur le dos et une position couchée sur le côté ;
dans lequel le corps en mousse est défini par une première section (1000) construite pour être utilisée lorsqu'un utilisateur se trouve dans la position couchée sur le dos, et une paire de deuxièmes sections adjacentes (900) destinées à être utilisées lorsqu'un utilisateur de trouve dans la position couchée sur le côté, la première section étant disposée entre les deuxièmes sections ;
dans lequel la première section (1000) du corps en mousse inclut lesdites première, deuxième et troisième régions (230, 240, 245) et les trois nervures (250) ; et
dans lequel les deuxièmes sections adjacentes du corps en mousse incluent une paire de découpes en forme d'arc (630) formées dans le bord avant, les découpes en forme d'arc étant espacées le long du bord avant pour recevoir une épaule d'un utilisateur couché sur le côté.

3. Oreiller (200, 800) selon les revendications 1 ou 2, dans lequel le corps en mousse (210) est composé de mousse viscoélastique, une tête et un cou d'un utilisateur étant ainsi arqués vers l'arrière au-dessus de la première région (230), supportés par le corps en mousse lorsque l'utilisateur se trouve dans une position couchée sur le dos, la tête étant au moins sensiblement contenue dans la deuxième région (240).

4. Oreiller (200, 800) selon la revendication 1, dans lequel les nervures (250) s'étendent le long de l'ensemble de la longueur (L2) du corps en mousse (210), d'un côté latéral (216) vers l'autre côté latéral (218).

5. Oreiller (200, 800) selon la revendication 1, dans lequel la première région (230) et définie par un premier rayon de courbure (R1), la deuxième région (240) étant définie par un deuxième rayon de courbure (R2) et la troisième région (245) étant définie par un troisième rayon de courbure (R3), le troisième rayon de courbure étant supérieur au premier rayon de courbure.

6. Oreiller (200, 800) selon la revendication 5, dans lequel le premier rayon de courbure (R1) correspond à environ 3,944 pouces (environ 100 mm) ; le deuxième rayon de courbure (R2) correspondant à environ 3,927 pouces (environ 99,75 mm) et le troisième rayon de courbure (R3) correspondant à environ 6,693 pouces (environ 170, mm).

7. Oreiller (800) selon la revendication 2, dans lequel la deuxième section (900) a une configuration étagée.

8. Oreiller (800) selon la revendication 7, dans lequel la deuxième section englobe une paire d'étagements (710, 722) agencés entre des première et deuxième parties planes, situées respectivement au niveau du bord avant et du bord arrière de l'oreiller, et dans lequel les première et deuxième parties planes sont optionnellement parallèles l'une à l'autre.

9. Oreiller (800) selon la revendication 8, dans lequel la paire d'étagements inclut un premier étagement (710) présentant une première pente et un deuxième étagement (722) présentant une deuxième pente, la première pente étant supérieure à la deuxième pente et étant agencée plus près du bord arrière, dans lequel une longueur du deuxième étagement est optionnellement supérieure à une longueur du premier étagement.

10. Oreiller (800) selon la revendication 2, dans lequel une paroi biseautée interne (1005) est formée au niveau d'une interface entre la première section (1000) et chaque deuxième section (900), la paroi biseautée interne ayant une hauteur variable, mesurée du bord arrière vers le bord avant.
